# EUROPEAN PATENT APPLICATION

(11) **EP 0 673 644 A1**
(43) Date of publication of application: **27.09.1995**
(21) Application number: 94830120.5
(22) Date of filing: 18.03.1994
(51) Int. Cl.: A61K 9/00

(54) **Process and plant for preparation of effervescent granules**

(71) Applicant: E-PHARMA S.p.A, I-38040 Trento Ravina (TN) (IT)
(72) Inventor: Carcano, Mario, CH-6805 Mezzovico (CH)
(74) Representative: Marietti, Giuseppe

(57) **Abstract**

Effervescent and soluble granulates containing an active principle are prepared according to a process of the type in which a mixture containing acid compounds and basic compounds are granulated in an air fluid bed, comprising the steps of : feeding to the fluid bed an air flow having total moisture content within the range from 0 to 1.0 g/mc; enbulizing water or an aqueous solution on the mixture, to effect granulation, evaporating the nebulized water from the forming granules before an uncontrolled reaction of effervescence occurs, but after actual granulation; continuing atomization and spraying of the water until required granulate size is reached, and essiccating the granulate to a moisture content of 0 to 1.0% by weight.

## Description

The present invention relates to a process and plant for the production of effervescent granulate. In particular, the present invention relates to a process and plant for the production of pharmaceutical formulations in the form of effervescent granulate, preferably soluble, as well as to the pharmaceutical formulations obtained therefrom, both in the form of granulate and in the form of tablets compressed from such granulate.

Effervescent granulate, both in the form of granulate and in the form of tablets compressed from such granulate, has been known to the art for some time and is used for the oral administration of such active principles as acetyl salicylic acid, ascorbic acid, paracetamol, ketoprofen, ibuprofen, carnitine, vitamin supplements, etc.

The increased attention being given to this form of administration is due to the many advantages it has over traditional tablets, such as, for example, greater bio-availability, ease of ingestion, and absence of any substantial intestinal-wall localized attack phenomena.

There are substantially two types of process used to produce the effervescent granulate. According to the first method, described in the *Journal of Pharmaceutical Sciences, 53, (1964), 1524-25,* the acidic and basic components are mixed with the active principle and additives (sweeteners and the like) in a fluid bed.

Granulation is achieved by spraying the mixture in the fluid bed with distilled water or an aqueous solution of diphosphate; the water causes an effervescent reaction which expands the granules in formation and binds other particles of the solid mixture until the required granule size is reached.

Such a process was adopted in, for example, Application No. EP-A-0233853, where water was added to mixture of citric acid and alkaline carbonates or bicarbonates to prepare an effervescent formulation; the mixture was reacted until carbon dioxide was given off equal to 23-54% of the mixture potential.

However, this is difficult to achieve since the process parameters to be controlled are numerous and the results are not very repeatable. In many cases the effervescent reaction cannot be limited to the initial granule layer and often spreads uncontrollably to all the mixture in the fluid bed.

Later technique returned to and in some cases modified previously known methods, i.e. granulation by alcohol solution.

An exhaustive examination of many known processes was included in Application No. EP-A-0233853 and is included herein for reference.

Another known technique is the separate granulation of the acidic and basic components, and mixing them at the end in anhydrous form. This technique is described in Application No. EP-A-0369228. The disadvantage of this technique is clearly its greater production cost.

Furthermore, all known techniques require the addition of binders to the components of the effervescent system to produce satisfactory granules. Further additives are required for the compression of the granules into tablets.

The plant required to produce the granulate also reflects the above problems; it consists essentially of a fluid bed connected to an air source fitted with a heater and filter, and an outlet for the air. Internally, the fluid bed (usually column-type) has a number of nozzles to spray water or, more often, water-alcohol solution.

One aim of the present invention is to provide a process for the preparation of effervescent soluble granulate, in particular containing one or more active principles, which allows a mixture of the acid and base components of the effervescent system to be granulated in a reliable and repeatable way using water in place of alcohol solutions. Another aim of the present invention is to provide a plant to operate the above mentioned process.

Such aims are achieved by means of the present invention which provides a process for the production of effervescent granulate according to Claim 1.

It has been surprisingly found that if the fluid bed containing the mixture of the acid and base components of the effervescent system is fed with an airstream containing from 0 to 1.0 g/m³ humidity and more generally from 0.1 to 1.0 g/m³, control of the entire process becomes quite easy, and the remaining parameters can be fixed with greater tolerance than is known in the art.

Furthermore, the process is perfectly repeatable and reproduceable and gives stable products.

It is maintained, without giving a complete scientific explanation of the process according to the present invention, that the air stream entering the fluid bed, because of its low moisture content, substantially inhibits the reaction between the acid and carbonates in the mixture and either stops it or prevents it from proceeding in any appreciable way. The effervescent reaction can be monitored by measuring the evolved carbon dioxide in the air flowing out of the fluid bed.

According to an aspect of the present invention the moisture content of the air entering the fluid bed is between 0.2 to 0.8 g/m³ and preferably between 0.4 to 0.6 g/m³. The air is heated during the granulation and drying phase and cooled during the final phase of the process, before the removal of the granulate.

According to another aspect of the present invention the pressure in the fluid bed is maintained at below atmospheric pressure.

According to a further aspect of the present invention, the product formed is packed in an atmosphere with controlled air moisture content, preferably below 20%.

According to another aspect of the present invention, the granulation time is optimized by spraying a quantity of atomized water into the fluid bed equal to that weight of water which is sufficient to give granulation without the effervescent reaction, or with a very reduced reaction, and in any case such as to keep the evolved CO₂ below 20% of the potential in the reaction.

This balance is maintained by considering the quantity of water already present in the starting materials and in the moisture-content of the air entering and leaving the fluid bed, and by taking into account the water formed by the effervescent reaction inside the mixture itself. The latter parameter can be detected by measuring the quantity of CO₂ exiting from the fluid bed. The quantity of water required for granulation can be calculated, preferably by microprocessor or other computerized means, from this measurement.

The present invention also relates to a plant for the production of the effervescent granulate, characterized according to Claim 9.

According to a preferred aspect of the present invention, the means of dehumidifying the air comprise first dehumidification means of a physical type which reduces the moisture content to about 4 - 5 g/m³, and second dehumidification means involving desiccation mixtures or compounds which reduce the moisture content to below 1 g/m³.

According to another aspect of the present invention, the second dehumidification means comprise rotating elements which successively present the same area of desiccating material to the partially desiccated airflow from the first dehumidification means, then to a second airflow at a temperature greater than 100°C and usually 120°C, and lastly to a third flow of cold air from the first dehumidification means.

According to another aspect of the present invention, the plant comprises means of heating or cooling the dried air so that the fluid bed is supplied with air at the most suitable temperature for each step of the process in the fluid bed.

According to another aspect of the present invention, the plant comprises detector means located between the dehumidifier and the fluid bed to measure the moisture content of the incoming airflow, and downstream of the said fluid bed to measure the moisture content of the outgoing air. As will be better explained below, this arrangement is used to control the process (together with the CO₂ detectors) as well as to verify that the drying of the granulate is complete.

According to another aspect of the present invention, the plant comprises computerized means of optimizing the process times on the basis of the amount of water entering and leaving the fluid bed, as described above.

The present invention relates furthermore to the effervescent granulate produced by the process described above, such granulate being characterized by having a moisture content of less than 0.8% by weight and preferably within the range of 0.4 - 0.6% by weight.

The effervescent granulate according to the present invention is preferably of a type suited to pharmaceutical use, containing therefore one or more active principles.

As mentioned above, the process and plant according to the present invention have multiple advantages. The present invention solves all the problems of the state of the art, allowing the granulation of the components forming the effervescent system in a single batch, in very short time (about 30 to 40 minutes for 400 kg batches), and in a totally reliable and repeatable way. The fast production time constitutes a considerable economic advantage, which allows the higher cost of plant compared to the traditional plant used until now to be recovered quickly.

Another advantage is that the product obtained according to the present invention is stable over time.

Another advantage is the high yield of the process, immediately obvious from the small difference between the weight of materials entering the fluid bed and the weight of effervescent granulate being removed from the same.

The present invention will now be described in more detail with reference to the attached drawings which are illustrative but not limiting, and where:
- Fig. 1 is a scheme of the plant according to the present invention;
- Fig. 2 is an enlarged view of the preferred means of dehumidification, and
- Fig. 3 is a chart of the operational control of the process with optimization of the water balance.

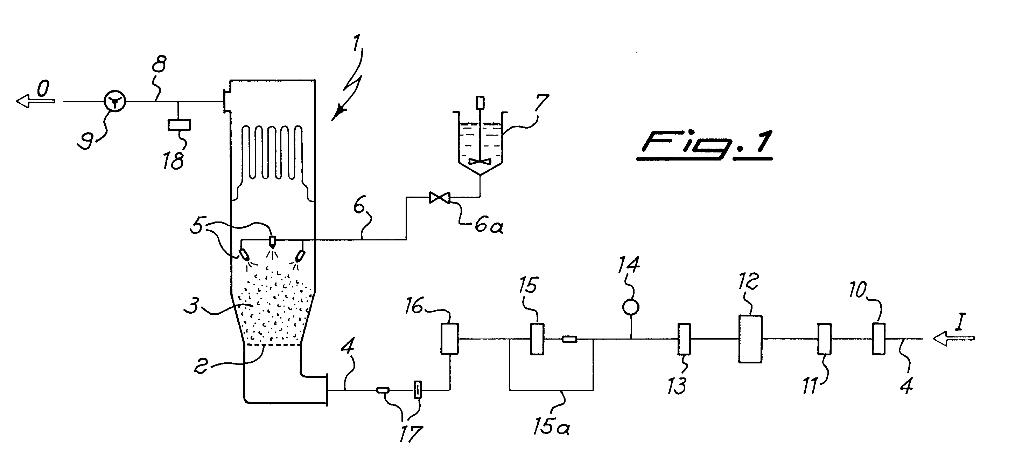

The plant shown in fig. 1 comprises a fluid bed granulation device 1, provided with a multi-airjet membrane 2, above which the mixture 3 of acidic and basic components of the effervescent system together with any additives and active principles are suspended by a continuous flow of air from the supply line 4.

Atomizer mist nozzles 5 are positioned inside the fluid-bed column 1 to spray demineralised water onto the mixture 3 suspended by the airflow. The demineralised water may contain one or more additives to assist granulation and/or any subsequent tablet formation. Such nozzles are known *per se* and in use for this purpose. Suitable nozzles would have a diameter between 0.8 and 1.8 mm. The nozzles 5 are connected in a known manner to the feeder pipe 6 for the water or aqueous solution coming from the mains supply or, preferably, a tank 7 which also serves as a mixer if there are additives to be added to the granulation water. A flow-rate regulation valve 6a is provided on the feeder pipe 6; the valve 6a may be manually operated or electronically operated by means of a computer.

The nozzles 5 are also connected to a supply of compressed air (not shown) to atomize the liquid in a manner already known in the art.

The direction of flow of the process air in the fluid bed column is indicated by the arrow I, which indicates the entry of the air along line 4 and by the arrow O, which indicates the air leaving the fluid bed column along line 8. A blower 9 is fitted to this line.

Upstream of the fluid bed column 1 air is drawn through two heat exchangers 10 and 11 which serve as first, or primary, dehumidification means and reduce the absolute moisture content of the air to 4 - 5 g/m³ and the temperature to about 5°C.

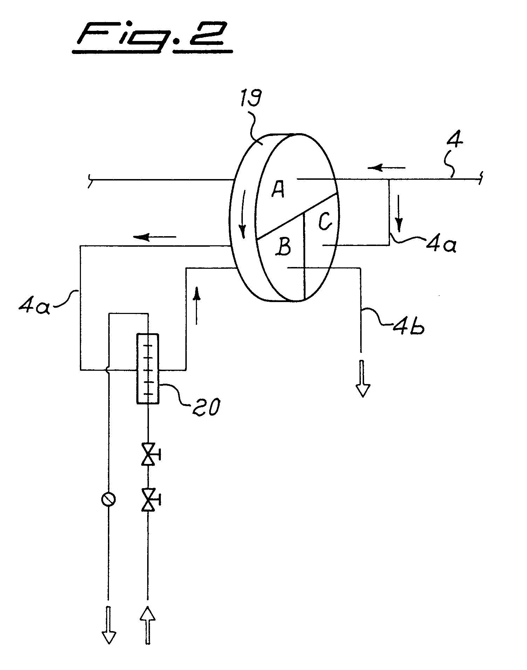

Then the air passes to second dehumidification means 12, where the the absolute moisture content of the air is reduced to below 1 g/m³ and generally between 0.1 and 1.0 g/m³. This means of dehumidification can be physical, similar to 10 and 11 e.g. a two stage direct expansion system. Alternatively, dehumidification means 12 may use water-absorption chemicals such as silica gel, lithium chloride or similar compounds. A preferred embodiment of dehumidifier 12 is shown in fig.2.

Downstream of the dehumidifier 12 is a means of cooling air 13 (i.e. a heat exchanger) which is required to cooln the air on exit from dehumidification means 12 for the final stage of the process, i.e after the desiccation of the granulate obtained from the mixture 3.

A moisture content detector 14 fitted to air line 4 below the heat exchanger 13 measures the absolute moisture content of the dehumidified air and sends a signal to a means of control, preferably electronic and computerized, which in turn regulates the operation of the dehumidification system.

A further heat exchanger 15 is used to raise the temperature of the dehumidified air to 70 - 90°C during the phases of granulation and desiccation of mixture 3 and has a by-pass line 15a. Filters 16 and flow regulators 17 are provided downstream heat exchanger 15. A further moisture content detector 18 is fitted to air exit line from the fluid bed column 1, to measure the total quantity of water in the air flow which passes through mixture 3.

In a preferred embodiment, the fluid bed 1 and the means of feeding the components of the mixture as well as the means of extraction of the granulate and the packaging of the same are maintained in a controlled environment, preferably at about 20% air moisture content.

The preferred embodiment of the means of dehumidification described in fig. 2 comprise a rotating element 19 containing silica gel or LiCl or similar desiccant capable of reducing the absolute moisture content of the air from 5 g/m³ to below 1 g/m³. Devices of this type are known in the art and are commercially available under the name Recusorb from the firm DST.

The air used for granulation preferably has an absolute moisture content of between 0.2 and 0.8 g/m³, preferably between 0.4 and 0.6 g/m³. There is a branch 4a of the air line 4 immediately upstream of the rotating element 19, supplying air cooled to 5°C and with a moisture content of about 5 g/m³. Branch line 4a supplies this flow of cool and dry air to zone C of the rotating element 19 whereas the main air line 4 supplies air to zone A. The cold and dry air passes through zone C of the rotating element 19 to a heat exchanger 20 (e.g. heated by steam) which raises the temperature of the air in line 4A to over 100°C and preferably to 120°C to remove all the water adsorbed by the desiccant material of the rotating element 19 while passing through zone C and A.

The humid air having passed through zone B of the rotating element exits to waste via exhaust line 4b.

The operation is as follows: initially, a flow of pre-cooled air is supplied to line 4 (arrow I) and passes through dehumidifiers 10 and 11, where the absolute moisture content of the air is reduced to 4 - 5 g/m³. From there the air flows to elements 12 or 19 where it is further dried to a total moisture content within the range of 0 to 1.0 g/m³, usually of 0.1 to 1.0 g/m³. Preferred ranges are 0.2 to 0.8 g/m³, and most preferably between 0.4 and 0.6 g/m³.

If the plant provides the use of rotating element 19, some of the air flow arriving along line 4 is diverted to pass through side-branch 4a. The ratio of flow rate in line 4 to flow rate in line 4a is between 10:1 and 9:5 preferably 8:3. The air flow passing through zone A of rotating element 19 is dried to the required moisture level and passes to the following section of line 4.

Zone A of the rotating element 19, loaded with the adsorbed moisture, is moved until it occupies the area formerly occupied by zone B of element 19, i.e. in front of line 4b. The desiccant material is here flushed by the airflow at 120°C, supplied by line 4a from the heat exchanger 20, that desorbs from said desiccant material the moisture previously adsorbed, and that is discharged, with the desorbed moisture, to exhaust line 4b. At this stage, the same initial zone A of the rotating element 19 turns further to occupy the area formerly occupied by zone C, where the desiccant material of element 19 is cooled down by the cold airflow from the line 4a bled off from line 4. The moisture content of the airflow exiting the rotating element 19 can be regulated by controlling the speed of rotation of element 19.

The dried air leaving the rotating element 19 flows through heat exchanger 13, which is activated only if granulation and desiccation of the mixture 3 is complete and it is necessary to cool the granulate.

From heat exchanger 13 the air passes to heat exchanger 15 where it is heated, if necessary, to the temperature required by the particular step of the process, generally up to 70 - 90°C. The heated air then passes through filter 16 and is fed into the fluid bed column 1, where it evaporates the water sprayed onto the particles of the mixture 3 after they have begun to agglomerate into granules, but be fore an uncontrollable effervescent reaction can start between the components of the same mixture.

The fluid bed column 1 is located in a controlled humidity environment, preferably at 20%, in order to avoid any adsorption of air moisture by starting material and by granulate while loading the materials and unloading the granulate. Both the packaging and the working of the granulate is carried out in a controlled humidity environment.

As previously stated, the use of moisture-controlled air flow within the specified ranges allows the process to operate "securely" with respect to the other parameters of the process, such as amount and flow rate of the atomized water, temperature and flow rate of the air.

Preferred conditions are an amount of water from 2 to 6% by weight of the weight of the mixture, with a flow rate of 400 to 1000 ml/min. Obviously, these values may be varied depending on the physical characteristics of the components of the system.

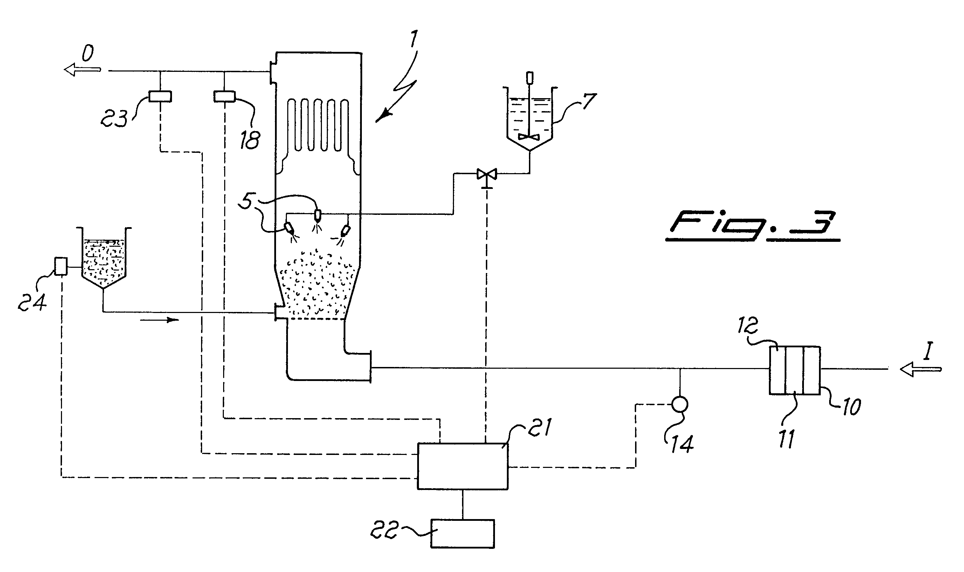

Figure 3 discloses a scheme of the process operation under computerized control, which control enables to optimize granulation time through a water balance of the process.

In more detail, the sensor 14 detects the moisture content of the air leaving the dehumidification means 10-12, and generates a signal which is sent to computerized control means 21. Similarly, the signals generated by sensor 18, detecting the moisture content of the air leaving the fluid bed column 1, and by the sensor 23, detecting the CO₂ content of the air leaving the fluid bed column 1, are sent to computer 21. Means 24 is also provided to detect the moisture content of the starting materials entering the fluid bed column 1. In fig.3 this means is shown, for the sake of simplicity, as a sensor; however, it is ob vious that it will actually be apparatus to analyze the moisture content of samples of the starting materials. The related data will be entered into the computer 21 in a known way, e.g. by keyboard. On the basis of the signals in arrival, the computer 21 will calculate the amount of water to be injected into the fluid bed column 1 and regulate the valve 6a on the water feeder line 6 to the atomizer nozzles accordingly. Furthermore, the CO₂ content of the air leaving the fluid bed column will give warning in real time of the start of any effervescent reaction between the components of the mixture before it can become uncontrollable. A further means of control 22 checks on the correct operation of the computer 21.

The moisture content of the air leaving the fluid bed column 1 is measured by detector 18. In this way a preferred value (that preferred for the effervescent granulate) for the moisture content of the air entering the fluid bed column 1 can be set; when the sensors 14 and 18 give the same value, the desiccation of the granulate is complete.

The present invention will now be described with reference to the following example.

### EXAMPLE 1

Production of effervescent tablets of ascorbic acid.

The following mixture was loaded into the fluid bed of a pilot plant arranged according to the present invention: 2.25 kg NaHCO₃; 2.95 kg KHCO₃; 1.08 kg Na₂CO₃; 1.60 Kg Citric Acid; 0.20 kg Aspartame (TM); 0.12 kg Orange Aroma and 1.80 kg Ascorbic Acid for a total of 10 kg. The mixture is suspended, in a known way, in a jet of dehumidified air with a moisture content of 0.6 g/m³, heated to a temperature of 65°C. The mixture is sprayed with 0.4 kg of atomized demineralized water at a flow-rate of 25 ml/min causing granulation.

At the end of granulation, the granulate is desiccated until the moisture content of the air leaving the fluid bed is the same level as the air entering it, i.e 0.6 g/m³. The granules are then cooled and unloaded in an environment with controlled moisture content of not more than 20%. The total process time is 35 minutes.

The granules are then compressed into tablets each weighing 2.5 g, corresponding to 0.45 g of active principle, which are packaged in the traditional manner, but in an environment with controlled moisture content.

## Claims

1. A process for the production of effervescent granulate, of the type in which a mixture containing acid compounds and basic compounds forming an effervescent system are granulated in a fluid bed over air, characterized by comprising the phases of:
supplying the said fluid bed with air having a total moisture content within the range from 0 to 1.0 g/m³;
spraying atomized water or aqueous solution on the said mixture to cause granulation, evaporating the sprayed water from the particles aggregating before an uncontrollable effervescent reaction starts, but after granulation;
continuing the said spraying and evaporation until the granules reach the desired size; and
desiccating the granules thus obtained until their moisture content is within the range from 0 to 1.0% by weight.

2. A process according to Claim 1, wherein the air supplied has a moisture content within the range from 0.2 to 0.8 g/m³, and preferably from 0.4 to 0.6 g/m³.

3. A process according to Claim 1 or 2, wherein the operating temperature is within the range of 30 to 90°C.

4. A process according to any of the preceding claims, wherein at least part of the granulation and desiccation is carried out at a pressure that is lower than atmospheric pressure.

5. A process according to any of the preceding claims, wherein an amount of water of 2 to 6% by weight of the total weight of the said mixture is atomized.

6. A process according to any of the preceding claims, wherein the water flow rate is within the range form 400 to 1000 ml/min.

7. A process according to any of the claims 1 - 6, wherein a water balance is operated for the process by measuring the moisture content of the air entering and leaving the said fluid bed, measuring the moisture content of the mixture, measuring the CO₂ content of the air leaving the said fluid bed and calculating the quantity of water required for the granulation of the said mixture from this data.

8. A process according to any of the preceding claims, wherein the said mixture comprises one or more pharmaceutically acceptable active principles.

9. A plant for the production of effervescent granulate, of the type comprising a fluid bed device for granulating a mixture containing acidic and basic compounds forming an effervescent system, and means of atomizing and spraying water or an aqueous solution in the said fluid bed, characterized by further comprising:
means of dehumidification to remove humidity from the air flow entering the said fluid bed to to a moisture content of said air flow within the range of 0 to 1.0 g/m³;
means of heating and cooling the said dehumidified air;
and means of measuring the moisture content of the said air.

10. A plant according to Claim 9, wherein the said means of dehumidification comprise first means for bringing the moisture content of the air to about 4 - 5 g/m³, and second means of dehumidification to reduce the said moisture content to a moisture content within the range of 0 to 1.0 g/m³.

11. A plant according to Claim 10, wherein the said second means comprise one or more elements containing desiccating compounds or mixtures.

12. A plant according to Claim 11, wherein the said elements are rotatable to present the same area of desiccant material successively to a cold, partially dehumidified airflow, a hot airflow with a temperature above 100°C and a cold, partially dehumidified airflow.

13. A plant according to one of claims 9 to 12, further comprising moisture detectors located between the second dehumidification means and the said fluid bed, as well as further moisture detectors located at the exit of the air from the said fluid bed.

14. A plant according to one of claims 9 to 13, further comprising CO₂ detectors to detect the CO₂ content of the air leaving the said fluid bed, detectors means to measure the moisture content of the said mixture, and computerized means of processing the signals in arrival from the said detectors and from the moisture content detectors for the air entering and leaving the said fluid bed, and of calculating the amount of water necessary to obtain the said granulation of the mixture.

15. An effervescent granulate as obtained from a process according to any of the Claims 1 to 8, characterized by having a total moisture content of below 1% and preferably between 0.1 and 0.8 % by weight.

16. A granulate according to Claim 15, in tablet form.
